# EUROPÄISCHE PATENTANMELDUNG

(11) **EP 0 841 058 A2**
(43) Veröffentlichungstag der Anmeldung: **13.05.1998**
(21) Anmeldenummer: 97115976.9
(22) Anmeldetag: 13.09.1997
(51) Int. Cl.: A61K 7/00, A61K 7/48, C09K 15/00, C11B 5/00

(54) **Phospholipidische Zusammensetzung, Verfahren zur Herstellung einer derartigen Zusammensetzung und Verwendung derselben**

(30) Priorität: 06.11.1996 DE 19645658
(71) Anmelder: RHONE-POULENC RORER GMBH, 50792 Köln (DE)
(72) Erfinder: Ghyczy, Miklos, Dr., 50933 Köln (DE)
(74) Vertreter: Lau-Loskill, Philipp, Dipl.-Phys.

(57) **Zusammenfassung**

Es werden eine phospholipidische Zusammensetzung sowie ein Verfahren zur Herstellung einer phospholipidischen Zusammensetzung, umfassend mindestens ein Phospholipid sowie einen Stabilisator, beschrieben, wobei die Zusammensetzung neben dem mindestens einen Phospholipid desweiteren als Stabilisator Phytinsäure, ein Salz der Phytinsäure und/oder ein Phytinsäurederivat enthält.

## Beschreibung

Die vorliegende Erfindung betrifft eine phospholipidische Zusammensetzung mit den Merkmalen des Oberbegriffs des Patentanspruchs 1, ein Verfahren zur Herstellung einer derartigen Zusammensetzung mit den Merkmalen des Oberbegriffs des Patentanspruchs 15 sowie die Verwendung der phospholipidischen Zusammensetzung.

Phospholipidische Zusammensetzungen sind seit langem bekannt und werden vielfach im Lebensmittelbereich, in der Tierernährung, im kosmetischen Bereich sowie im pharmazeutischen Bereich verwendet. Hierbei besteht jedoch das Problem, daß derartige phospholipidische Zusammensetzungen, die abhängig von der Konzentration an Phospholipiden und der jeweiligen Anwendung, flüssig, halbfest, wie insbesondere gelartig, cremeartig, pastös, oder fest sind, sehr leicht an der Luft und/oder beim Lagern einen Eigengeruch, insbesondere einen ranzigen Eigengeruch, entwickeln, was dementsprechend unerwünscht ist.

Um diese unerwünschte Geruchsveränderung von phospholipidischen Zusammensetzungen zu unterdrücken, ist es bekannt, die phospholipidischen Zusammensetzungen mit einem Stabilisator zu versehen, wobei derartige Stabilisatoren auf der Basis von Vitamin C, Vitamin E und/oder deren Derivaten aufgebaut sind. Desweiteren können phospholipidische Zusammensetzungen auch gemäß der DE 41 41 842 A1 durch Verwendung von N-Acyl-Phosphatidyl-ethanolaminen vor einer unerwünschten Oxidation geschützt werden.

Darüber hinaus schlägt die DE 40 21 082 A1 vor, zur Stabilisierung von phospholipidischen Zusammensetzungen Harnstoff, Monosaccharide oder Mischungen von Harnstoff mit Monosacchariden zu verwenden.

Der vorliegenden Erfindung liegt die Aufgabe zugrunde, eine phospholipidische Zusammensetzung der angegebenen Art zur Verfügung zu stellen, die auch bei einer längeren Lagerung selbst unter Luftzufuhr keine Geruchsveränderung erfährt.

Diese Aufgabe wird erfindungsgemäß durch eine phospholipidische Zusammensetzung mit den kennzeichnenden Merkmalen des Patentanspruchs 1 gelöst.

Die erfindungsgemäße phospholipidische Zusammensetzung umfaßt mindestens ein Phospholipid sowie einen Stabilisator, wobei die Zusammensetzung neben dem mindestens einen Phospholipid desweiteren als Stabilisator Phytinsäure, ein Salz der Phytinsäure und/oder ein Phytinsäurederivat enthält.

Überraschend konnte festgestellt werden, daß die erfindungsgemäße phospholipidische Zusammensetzung, die als Stabilisator Phytinsäure, ein Salz der Phytinsäure und/oder ein Phytinsäurederivat enthält, eine hervorragende Stabilität selbst bei einer Lagerung unter Luft aufweist, so daß auch nach einer längeren Lagerzeit von mehreren Monaten keine unerwünschte Geruchsveränderung bei der so stabilisierten phospholipidischen Zusammensetzung auftritt. Dies führt dazu, daß die erfindungsgemäße phospholipidische Zusammensetzung nicht, wie die bisher bekannten phospholipidischen Zusammensetzungen, unter Inertgas, so zum Beispiel Stickstoff, bei verringerten Temperaturen, so insbesondere Temperaturen um den Gefrierpunkt, gelagert werden muß. Dementsprechend lassen sich aus der erfindungsgemäßen Zusammensetzung auch bezüglich der Phospholipidkonzentration reproduzierbare Folgeprodukte, wie beispielsweise Creme, Gele, Salben, Dispersionen, halbfeste oder flüssige liposomale Zubereitungen, herstellen, da in der erfindungsgemäßen Zusammensetzung die Phospholipidkonzentration auch bei einer längeren Lagerung konstant bleibt.

Eine erste Ausführungsform der erfindungsgemäßen Zusammensetzung sieht vor, daß die Zusammensetzung als Stabilisator ein Salz der Phytinsäure, insbesondere ein Erdalkali- und/oder Alkalisalz der Phytinsäure, enthält.

Vorzugsweise weist die erfindungsgemäße phospholipidische Zusammensetzung als Stabilisator ein Calcium- und/oder Magnesiumsalz der Phytinsäure auf, wobei auch ein Calcium-/Magnesium-Mischsalz eine ausgezeichnete Stabilisierung der phospholipidischen Zusammensetzung bewirkt.

Bei der zuvor genannten Phytinsäure, dem Salz der Phytinsäure und/oder dem Derivat der Phytinsäure kann es sich entweder um ein synthetisch hergestelltes Produkt oder um ein aus Samenschalen und/oder der Aleuronschicht von Getreide durch Extraktion mit einem wäßrigen System und/oder einem entsprechenden Lösungsmittel handeln. Typische Getreidearten, aus denen die Phytinsäure, das Salz der Phytinsäure und/oder das Derivat der Phytinsäure gewonnen werden, sind Weizen, Hafer, Mais, Reis, Roggen, Gerste und/oder Hirse.

Hinsichtlich der in der erfindungsgemäßen Zusammensetzung vorhandenen Derivate der Phytinsäure sind insbesondere Ester, Ether, Amide, Amine und/oder deren Salze zu nennen.

Bezüglich des Gewichtsverhältnisses von dem mindestens einen Phospholipid zu der Phytinsäure, der Salz der Phytinsäure und/oder dem Derivat der Phytinsäure in der erfindungsgemäßen Zusammensetzung ist festzuhalten, daß sich dieses Gewichtsverhältnis einerseits nach der gewünschten Stabilisierung und andererseits nach der jeweiligen Verwendung der erfindungsgemäßen phospholipidischen Zusammensetzung richtet.

Üblicherweise variiert in der erfindungsgemäßen Zusammensetzung das Gewichtsverhältnis von dem mindestens einen Phospholipid zu dem zuvor beschriebenen Stabilisator zwischen 1:0,08 bis 1:0,002, vorzugsweise zwischen 1:0,04 und 1:0,003. Ist eine extrem lange lagerfähige phospholipidische Zusammensetzung erwünscht, so weist diese Ausführungsform einen relativ hohen Gehalt an Stabilisator auf. Das gleiche gilt für solche Ausführungsformen der erfindungsgemäßen Zusammensetzung, die zu Sonnenschutzprodukten weiterverarbeitet werden. Mit anderen Worten läßt sich somit durch Variation der Menge der zuvor genannten Stabilisatoren eine gewünschte Lagerstabilität der erfindungsgemäßen Zusammensetzung einstellen.

Zu den in der erfindungsgemäßen Zusammensetzung enthaltenen Phospholipid ist festzuhalten, daß es sich hierbei insbesondere um ein aus Pflanzen und vorzugsweise aus Soja-Bohnen isoliertes Phospholipid oder Phospholipidgemisch handelt.

Besonders geeignet ist es, wenn das in der erfindungsgemäßen Zusammensetzung vorhandene Phospholipidgemisch wenigstens 70 Gew.% 1,2-Diacylglycero-3-Phosphatidylcholin aufweist.

Bei einer weiteren Ausführungsform der erfindungsgemäßen Zusammensetzung umfaßt das in der Zusammensetzung enthaltene Phospholipidgemisch mindestens 76 Gew.% ± 3 Gew.% 1,2-Diacylglycero-3-Phosphatidylcholin und 3 Gew.% ± 3 Gew.% Lysophosphatidylcholin.

Eine andere, ebenfalls geeignete Ausführungsvariante der erfindungsgemäßen Zusammensetzung sieht vor, daß hierbei das Phospholipidgemisch ein phosphatidylcholinreiches Phospholipidgemisch ist und vorzugsweise 93 Gew.% ± 3 Gew.% 1,2-Diacylglycero-3-Phosphatidylcholin und insbesondere mindestens 96 Gew.% 1,2-Diacylglycero-3-Phosphatidylcholin enthält, wobei als weiteres Phospholipid dann zusätzlich insbesondere noch 3 Gew.% ± 3 Gew.% Lyso-Phosphatidylcholin vorhanden ist.

Neben den zuvor beschriebenen Phosphatidylcholinen kann die erfindungsgemäße Zusammensetzung insbesondere noch 1,2-Diacylglycero-3-phosphoethanolamin, 1,2-Diacylglycero-3-phosphoinositol, 1,2-Diacylglycero-3-phosphoserin, 1,2-Diacylglycero-3-phosphoglycerol und 1,2-Diacylglycero-3-phosphat als weitere Phospholipide, abhängig von dem jeweiligen Ausgangsmaterial und den angewandten Isolierungs- und Reinigungsverfahren, enthalten.

Bei einer weiteren bevorzugten Ausführungsform weist die erfindungsgemäße Zusammensetzung ein solches Phospholipidgemisch auf, bei dem die Acylreste der im Gemisch enthaltenen Phospholipide und insbesondere die Acylreste des im Gemisch vorgesehenen Phosphatidylcholins zu
- 61 - 73: Gew.% aus dem Linolsäurerest,
- 10 - 14: Gew.% aus dem Palmitinsäurerest,
- 8 - 12: Gew.% aus dem Ölsäurerest,
- 4 - 6: Gew.% aus dem Linolensäurerest,
- 3 - 5: Gew.% aus dem Stearinsäurerest und/oder
- 2: Gew.% aus anderen Fettsäureresten
bestehen. Bei einem derartigen oder den nachfolgend noch konkret beschriebenen Phospholipidgemisch bzw. Phosphatidylcholinen treten insbesondere die vorstehend bei der erfindungsgemäßen Zusammensetzung beschriebenen Vorteile besonders hervor, da derartige Phospholipide bzw. Phosphatidylcholine aufgrund ihres hohen Anteils an ungesättigten Fettsäureresten selbst bei hoher Reinheit sehr schnell dazu neigen, bei Lagerung selbst bei Ausschluß von Luftsauerstoff schnell einen in der Intensität zunehmenden Eigengeruch entwickeln.

Bei einer weiteren Ausführungsform der erfindungsgemäßen Zusammensetzung weist diese ein Phospholipidgemisch bzw. ein Phosphatidylcholin auf, bei dem die 1-Acylreste der im Gemisch enthaltenen Phospholipide bzw. die 1-Acylreste des Phosphatidylcholins zu
- 45 - 61: Gew.% aus dem Linolsäurerest,
- 19 - 26: Gew.% aus dem Palmitinsäurerest,
- 8 - 12: Gew.% aus dem Ölsäurerest,
- 4 - 6: Gew.% aus dem Linolensäurerest,
- 6 - 9: Gew.% aus dem Stearinsäurerest und/oder
- 2: Gew.% aus anderen Fettsäureresten
bestehen.

Ebenso kann die erfindungsgemäße Zusammensetzung auch ein solches Phospholipidgemisch bzw. Phosphatidylcholin aufweisen, dessen Acylreste in der 1-Stellung die zuvor angegebenen Acylreste oder andere Acylreste aufweist und bei denen die Acylreste in der 2-Stellung zu
- 77 - 85: Gew.% aus dem Linolsäurerest,
- 1 - 2: Gew.% aus dem Palmitinsäurerest,
- 8 - 12: Gew.% aus dem Ölsäurerest,
- 4 - 6: Gew.% aus dem Linolensäurerest,
- 0 - 1: Gew.% aus dem Stearinsäurerest und/oder
- 2: Gew.% aus anderen Fettsäureresten
bestehen.

Bei einer anderen Ausführungsform der erfindungsgemäßen Zusammensetzung weist dieses als Phospholipidgemisch ein aus Pflanzen, vorzugsweise aus Soja-Bohnen, isoliertes flüssiges Phospholipidgemisch auf, wobei dieses flüssige Phospholipidgemisch dann mindestens 40 Gew.% 1,2-Diacylglycero-3-Phosphatidylcholin enthält. Desweiteren sind in diesem flüssigen Phospholipidgemisch noch die üblichen Begleitphospholipide sowie Öle, vorzugsweise pflanzliche Öle, wie insbesondere Sonnenblumenöl, Distelöl, Avocadoöl, Mandelöl, Sojaöl, Rhizinusöl, Erdnußöl, Weizenkeimöl, Karottenöl, Haselnußöl, Palmkernöl, Sesamöl, Olivenöl, Walnußöl, Maiskeimöl o.dgl., vorhanden.

Als weiterer wesentlicher Vorteil der zuvor beschriebenen Ausführungsformen der erfindungsgemäßen Zusammensetzung ist festzuhalten, daß durch Zusatz der vorstehend genannten Stabilisatoren zu der phospholipidischen Zusammensetzung auch die aus dieser stabilisierten phospholipidischen Zusammensetzung hergestellten Folgeprodukte, so insbesondere die bereits vorstehend genannten Creme, Gele, Salben, Dispersionen, halbfeste oder flüssige liposomale Zubereitungen, stabilisiert werden, so daß auf den Zusatz eines separaten Stabilisators in diesen Folgeprodukten verzichtet werden kann.

Die vorliegende Erfindung betrifft desweiteren ein Verfahren zur Herstellung der zuvor beschriebenen erfindungsgemäßen Zusammensetzung.

Diese Aufgabe wird erfindungsgemäß durch ein Verfahren mit den kennzeichnenden Merkmalen des Patentanspruchs 15 oder mit den kennzeichnenden Merkmalen des Patentanspruchs 22 gelöst.

Das erfindungsgemäße Verfahren zur Herstellung der zuvor beschriebenen erfindungsgemäßen phospholipidischen Zusammensetzung sieht vor, daß das mindestens eine Phospholipid bzw. das Phospholipidgemisch in einem Lösungsmittel oder Lösungsmittelgemisch zunächst gelöst oder dispergiert wird. Anschließend wird diese Lösung bzw. diese Dispersion des Phospholipids bzw. des Phospholipidgemisches in eine Lösung oder Dispersion des zuvor beschriebenen Stabilisators eingebracht, so daß dann die so hergestellte Mischung unter Ausbildung der erfindungsgemäßen phospholipidischen Zusammensetzung schonend getrocknet, insbesondere sprühgetrocknet oder gefriergetrocknet, wird.

Das erfindungsgemäße Verfahren weist den Vorteil auf, daß es relativ einfach durchzuführen ist. Dies hängt damit zusammen, daß bei dem erfindungsgemäßen Verfahren lediglich zwei Lösungen bzw. Dispersionen miteinander vermischt werden, so daß hiernach nur noch diese Mischung getrocknet wird und somit die erfindungsgemäße Zusammensetzung anfällt. Selbstverständlich ist es bei dem zuvor beschriebenen erfindungsgemäßen Verfahren auch möglich, die Lösung oder Dispersion des Stabilisators in die Lösung bzw. Dispersion des Phospholipids bzw. des Phospholipidgemischs einzubringen und hiernach diese Mischung dann schonend zu trocknen, insbesondere sprühzutrocknen oder gefrierzutrocknen.

Grundsätzlich ist bezüglich der Auswahl des bei dem erfindungsgemäßen Verfahren eingesetzten Lösungsmittels bzw. Lösungsmittelgemischs festzuhalten, daß das Lösungsmittel bzw. Lösungsmittelgemisch für das Phospholipid bzw. für das Phospholipidgemisch und das Lösungsmittel bzw. das Lösungsmittelgemisch für den Stabilisator entsprechend derart aufeinander angepaßt werden, daß diese beiden Lösungsmittel bzw. diese beiden Lösungsmittelgemische auch untereinander mischbar sind. Vorzugsweise werden bei dem erfindungsgemäßen Verfahren als Lösungsmittel bzw. Lösungsmittelgemisch Wasser und/oder ein Alkohol bzw. ein Alkoholgemisch, insbesondere Ethanol, Propanol-1 und/oder Propanol-2, verwendet.

Eine besonders geeignete und vorteilhafte Ausführungsform des erfindungsgemäßen Verfahrens sieht vor, daß hierbei eine alkoholische Lösung des Phospholipids bzw. des Phospholipidgemisches, insbesondere eine ethanolische Lösung des Phospholipids bzw. Phospholipidgemischs, hergestellt wird und daß dann diese alkoholische Lösung in eine wäßrige Dispersion oder in eine wäßrige Lösung des Stabilisators eingerührt oder injiziert wird. Unter Wasser im Rahmen der vorliegenden Beschreibung werden alle wäßrigen System verstanden, so insbesondere destilliertes Wasser, entionisiertes Wasser, wäßrige Salzlösungen oder wäßrige Puffersysteme, so beispielsweise Phosphatpuffer.

Bezüglich der Konzentration des Phospholipids bzw. des Phospholipidgemisches in der alkoholischen Lösung ist festzuhalten, daß üblicherweise bei dem erfindungsgemäßen Verfahren diese alkoholische Lösung zwischen 70 Gew.% und 85 Gew.% des Phospholipids bzw. des Phospholipidgemisches enthält. Dementsprechend weist diese alkoholische Phospholipidlösung dann zwischen 30 Gew.% und 15 Gew.% Alkohol, insbesondere Ethanol, auf.

Eine andere Ausführungsform des erfindungsgemäßen Verfahrens sieht vor, daß hierbei eine wäßrige Dispersion des Phospholipids hergestellt wird und daß diese wäßrige Dispersion des Phospholipids dann in die wäßrige Dispersion des Stabilisators eingerührt oder injiziert wird. Diese Variante des erfindungsgemäßen Verfahrens weist den Vorteil auf, daß völlig auf Alkohole als Lösungsmittel verzichtet wird, so daß diese Variante des erfindungsgemäßen Verfahrens dann bevorzugt wird, wenn der entsprechende Hersteller über keine dementsprechenden Anlagen verfügt, die die Verarbeitung von brennbaren Lösungsmittel gestattet.

Vorzugsweise enthält die zuvor beschriebene wäßrige Dispersion zwischen 5 Gew.% und 20 Gew.%, insbesondere zwischen 8 Gew.% und 15 Gew.%, des Phospholipids bzw. des Phospholipidgemisches.

Wird bei dem erfindungsgemäßen Verfahren mit einer wäßrigen Dispersion oder einer wäßrigen Lösung der vorstehend genannten Stabilisatoren gearbeitet, so empfiehlt es sich, daß hierbei diese wäßrige Dispersion bzw. Lösung des Stabilisators zwischen 10 Gew.% und 30 Gew.%, insbesondere zwischen 15 Gew.% und 20 Gew.% des Stabilisators enthält. Durch Abstimmung der Menge dieser Lösung bzw. Dispersion auf die Menge der Phospholipidlösung bzw. -dispersion läßt sich dann besonders einfach bei der Herstellung der erfindungsgemäßen Zusammensetzung ein Gewichtsverhältnis von Phospholipid bzw. Phospholipidgemisch zu Stabilisator von 1:0,08 bis 1:0,002, insbesondere zwischen 1:0,04 und 1:0,003, einstellen.

Sollen bei den zuvor beschriebenen Ausführungsvarianten des erfindungsgemäßen Verfahrens noch weitere Substanzen, so insbesondere die vorstehend genannten Öle, hinzugefügt werden, so daß dementsprechend die so hergestellte erfindungsgemäße Zusammensetzung dann diese weiteren Substanzen enthält, so kann dies grundsätzlich auf drei unterschiedlichen Wegen bewerkstelligt werden.

So sieht die erste Möglichkeit vor, daß hierbei diese weiteren Substanzen der Lösung bzw. der Dispersion des Phospholipids zugesetzt werden, während bei der zweiten Möglichkeit diese weiteren Substanzen zu der Lösung bzw. der Dispersion des Stabilisators zugegeben werden. Ebenso kann als dritte Möglichkeit eine separate Lösung bzw. Dispersion dieser weiteren Substanzen hergestellt werden, wobei diese Lösung bzw. Dispersion dann der Mischung aus der Phospholipidlösung bzw. der Phospholipiddispersion und der Stabilisatorlösung bzw. Stabilisatordispersion zugesetzt wird.

Eine weitere Möglichkeit zur Herstellung der erfindungsgemäßen Zusammensetzung sieht vor, daß hierbei ein flüssiges Phospholipid bzw. ein flüssiges Phospholipidgemisch hergestellt wird, derart, daß das Phospholipid bzw. das Phospholipidgemisch in einem Öl, insbesondere den zuvor beschriebenen Ölen oder Ölgemischen, aufgenommen wird. Anschließend wird das in dem Öl aufgenommen Phospholipid bzw. Phospholipidgemisch mit der Dispersion bzw. Lösung des Stabilisators unter Ausbildung der erfindungsgemäßen Zusammensetzung vermischt.

Selbstverständlich ist es möglich, in Abwandlung dieses zuvor beschriebenen Verfahrens das Phospholipid bzw. Phospholipidgemisch dadurch zu verflüssigen, daß man es nicht in einem Öl aufnimmt sondern stattdessen das Phospholipid bzw. Phospholipidgemisch selbst durch Anwendung erhöhter Temperaturen aufschmilzt und das so aufgeschmolzene Phospholipid bzw. Phospholipidgemisch mit dem festen Stabilisator oder einer Lösung bzw. Dispersion des Stabilisators vermischt.

Bezüglich der Verwendung der erfindungsgemäßen Zusammensetzung ist festzuhalten, daß die erfindungsgemäße Zusammensetzung insbesondere zur Herstellung von kosmetischen Mitteln oder pharmazeutischen Mitteln verwendet wird. Bei den kosmetischen Mitteln kann die erfindungsgemäße Zusammensetzung vorzugsweise zur Herstellung von Hautbehandlungsmitteln, insbesondere den üblichen Salben, Cremes, Lotion, Milch, Salbengrundstoffe und/oder Cremegrundstoffe verwendet werden.

Im pharmazeutischen Bereich liegt insbesondere das Hauptanwendungsgebiet der erfindungsgemäßen Zusammensetzung in der Herstellung von halbfesten, gelartigen oder festen Grundstoffen, so zum Beispiel von entsprechenden Lotionen, Cremen, Gelen, Salben, die nach Zusatz von geeigneten Wirkstoffen dann topisch angewendet werden. Ebenso können aus der erfindungsgemäßen Zusammensetzung auch flüssige Formulierungen, insbesondere liposomenhaltige Formulierungen, erstellt werden, wobei derartige flüssige Formulierungen dann zusätzlich noch einen oder mehrere Wirkstoffe enthalten.

Vorteilhafte Weiterbildungen der erfindungsgemäßen Zusammensetzung sowie des erfindungsgemäßen Verfahrens sind in den Unteransprüchen angegeben.

Die erfindungsgemäße Zusammensetzung und das erfindungsgemäße Verfahren werden nachfolgend anhand von Ausführungsbeispielen näher erläutert.

### Ausführungsbeispiel 1

800 g eines Phospholipids, das mindestens 96 Gew.% Phosphatidylcholin sowie andere Begleitphospholipide enthält, wurden in 200 g Alkohol gelöst. Die so hergestellte Lösung wurde als Lösung 1 bezeichnet.

4 g Phytinsäure wurden in 800 g Wasser gelöst. Die so hergestellte Lösung wurde als Lösung 2 bezeichnet.

100 g der Lösung 1 wurden mit 200 g der Lösung 2 vermischt, wobei die Vermischung während 5 Minuten mit Hilfe eines schnell laufenden Rührwerkes (1.000 U/min) erfolgte. Anschließend wurde die entstandene flüssige Mischung einer üblichen Sprühtrocknung unterworfen.

Hierbei entstand eine phospholipidische Zusammensetzung I, bei der das Gewichtsverhältnis von Phospholipid zu Stabilisator 1:0,0025 betrug.

### Ausführungsbeispiel 2

50 g der Lösung 1 wurden mit 200 g der Lösung 2, hergestellt jeweils nach dem Ausführungsbeispiel 1, unter den dort genannten Bedingungen vermischt.

Die so entstandene Zusammensetzung wies nach Sprühtrocknung ein Gewichtsverhältnis von Phospholipid zu Stabilisator von 1:0,005 auf.

### Ausführungsbeispiel 3

50 g der Lösung 1 wurden mit 400 g der Lösung 2, hergestellt nach Ausführungsbeispiel 1, unter den dort genannten Bedingungen vermischt. Nach der Sprühtrocknung wies die so hergestellte Zusammensetzung III ein Gewichtsverhältnis von Phospholipid zu Stabilisator von 1:0,01 auf.

### Ausführungsbeispiel 4

50 g der Lösung 1 wurden mit 800 g der Lösung 2 unter den unter dem Ausführungsbeispiel 1 genannten Bedingungen vermischt. Nach der Sprühtrocknung wies die Zusammensetzung IV ein Mischungsverhältnis von Phospholipid zu Stabilisator von 1:0,02 auf.

### Ausführungsbeispiel 5

100 g der Lösung 1 wurden mit 200 g der Lösung 2 unter den im Ausführungsbeispiel 1 genannten Bedingungen vermischt. Zu der Mischung wurde unter Rühren 3 g eines Sonnenblumenöls getropft, wobei die hierbei entstehende Dispersion/Emulsion für weitere 10 Minuten gerührt wurde. Die so entstandene Mischung wurde sprühgetrocknet. Hiernach entstand eine Zusammensetzung, die nachfolgend auch als Zusammensetzung V bezeichnet ist und bei der das Gewichtsverhältnis von Phospholipid zu Stabilisator bei 1:0,0025 lag.

### Ausführungsbeispiel 6

80 g des im Ausführungsbeispiel 1 konkretisierten Phospholipids wurden in 20 g Ethanol gelöst. In diese alkoholische Lösung wurden 2 g Tocopherol eingerührt. Anschließend erfolgte eine Sprühtrocknung. Die hierbei anfallende Zusammensetzung VI wies ein Verhältnis von Phospholipid zu Stabilisator von 1:0,025 auf.

Von den vorstehend genannten Zusammensetzungen I bis VI wurde ein Alterungstest dahingehend durchgeführt, daß der Eigengeruch der Zusammensetzungen von vier Personen unabhängig voneinander sowohl unmittelbar nach der Herstellung sowie nach einer Lagerung von einer Woche, einem Monat, drei Monaten und sechs Monaten beurteilt wurde. Zur Objektivierung wurde bei dieser sensorischen Geruchsbeurteilung jeweils im Vergleich eine frisch zubereitete Probe der jeweiligen Zusammensetzung mitbeurteilt. Die Geruchsbildung wurde nach einer willkürlichen Klassifizierung, die die Noten 1 bis 4 umfaßt, vorgenommen, wobei die Note 1 keine Geruchsveränderung und die Note 4 einen starken, ranzigen Geruchseindruck wiedergibt. Die Note 2 bewertet eine geringe Geruchsveränderung, die Note 3 eine entsprechend stärkere Geruchsveränderung.

Die Ergebnisse dieser sensorischen Geruchsprüfung sind in der nachfolgenden Tabelle wiedergegeben.

**Tabelle 1**

| Zusammensetzung Nr. | unmittelbar nach der Herstellung | nach einer Woche | nach einem Monat | nach drei Monaten | nach sechs Monaten |
|---|---|---|---|---|---|
| I | 1 | 1 | 1 | 2 | 2 |
| II | 1 | 1 | 1 | 1 | 1 |
| III | 1 | 1 | 1 | 1 | 1 |
| IV | 1 | 1 | 1 | 1 | 1 |
| V | 1 | 1 | 1 | 1 | 1 |
| VI | 1 | 1 | 2 | 3 | 3 |

## Patentansprüche

1. Phospholipidische Zusammensetzung, umfassend mindestens ein Phospholipid sowie einen Stabilisator, dadurch gekennzeichnet, daß die Zusammensetzung neben dem mindestens einen Phospholipid desweiteren als Stabilisator Phytinsäure, ein Salz der Phytinsäure und/oder ein Phytinsäurederivat enthält.

2. Zusammensetzung nach Anspruch 1, dadurch gekennzeichnet, daß die Zusammensetzung als Stabilisator ein Salz der Phytinsäure, insbesondere ein Erdalkali- und/oder Alkalisalz der Phytinsäure, enthält.

3. Zusammensetzung nach Anspruch 1 oder 2, dadurch gekennzeichnet, daß die Zusammensetzung als Stabilisator ein Calcium- und/oder Magnesiumsalz der Phytinsäure aufweist.

4. Zusammensetzung nach einem der vorangehenden Ansprüche, dadurch gekennzeichnet, daß in der Zusammensetzung das Gewichtsverhältnis von dem mindestens einen Phospholipid zu dem Stabilisator zwischen 1:0,08 bis 1:0,002, insbesondere zwischen 1:0,04 und 1:0,003, variiert.

5. Zusammensetzung nach einem der vorangehenden Ansprüche, dadurch gekennzeichnet, daß das Phospholipid aus Pflanzen, vorzugsweise aus Soja-Bohnen, isoliert ist.

6. Zusammensetzung nach einem der vorangehenden Ansprüche, dadurch gekennzeichnet, daß das mindestens eine Phospholipid ein Phospholipidgemisch ist.

7. Zusammensetzung nach Anspruch 5 oder 6, dadurch gekennzeichnet, daß das Phospholipidgemisch wenigstens 70 Gew.% 1,2-Diacylglycero-3-Phosphatidylcholin aufweist.

8. Zusammensetzung nach einem der vorangehenden Ansprüche, dadurch gekennzeichnet, daß das Phospholipidgemisch mindestens
76 Gew.% ± 3 Gew.% 1,2-Diacylglycero-3-Phosphatidylcholin und
3 Gew.% ± 3 Gew.% Lyso-Phosphatidylcholin
enthält.

9. Zusammensetzung nach einem der vorangehenden Ansprüche, dadurch gekennzeichnet, daß das Phospholipidgemisch mindestens
93 Gew.% ± 3 Gew.% 1,2-Diacylglycero-3-Phosphatidylcholin und
3 Gew.% ± 3 Gew.% Lyso-Phosphatidylcholin
enthält.

10. Zusammensetzung nach einem der vorangehenden Ansprüche, dadurch gekennzeichnet, daß das Phospholipidgemisch desweiteren
1,2-Diacylglycero-3-phosphoethanolamin,
1,2-Diacylglycero-3-phosphoinositol,
1,2-Diacylglycero-3-phosphoserin,
1,2-Diacylglycero-3-phosphoglycerol und/oder
1,2-Diacylglycero-3-phosphat
enthält.

11. Zusammensetzung nach einem der vorangehenden Ansprüche, dadurch gekennzeichnet, daß die Zusammensetzung ein solches Phospholipidgemisch enthält, bei dem die Acylreste der im Gemisch enthaltenen Phospholipide zu
61 - 73 Gew.% aus dem Linolsäurerest,
10 - 14 Gew.% aus dem Palmitinsäurerest,
8 - 12 Gew.% aus dem Ölsäurerest,
4 - 6 Gew.% aus dem Linolensäurerest,
3 - 5 Gew.% aus dem Stearinsäurerest und/oder
2 Gew.% aus anderen Fettsäureresten
bestehen.

12. Zusammensetzung nach einem der vorangehenden Ansprüche, dadurch gekennzeichnet, daß die Zusammensetzung ein solches Phospholipidgemisch enthält, bei dem die 1-Acylreste der im Gemisch enthaltenen Phospholipide zu
45 - 61 Gew.% aus dem Linolsäurerest,
19 - 26 Gew.% aus dem Palmitinsäurerest,
8 - 12 Gew.% aus dem Ölsäurerest,
4 - 6 Gew.% aus dem Linolensäurerest,
6 - 9 Gew.% aus dem Stearinsäurerest und/oder
2 Gew.% aus anderen Fettsäureresten
bestehen.

13. Zusammensetzung nach einem der vorangehenden Ansprüche, dadurch gekennzeichnet, daß die Zusammensetzung ein solches Phospholipidgemisch enthält, bei dem die 2-Acylreste der im Gemisch enthaltenen Phospholipide zu
77 - 85 Gew.% aus dem Linolsäurerest,
1 - 2 Gew.% aus dem Palmitinsäurerest,
8 - 12 Gew.% aus dem Ölsäurerest,
4 - 6 Gew.% aus dem Linolensäurerest,
0 - 1 Gew.% aus dem Stearinsäurerest und/oder
2 Gew.% aus anderen Fettsäureresten
bestehen.

14. Zusammensetzung nach einem der Ansprüche 1 bis 6, dadurch gekennzeichnet, daß die Zusammensetzung ein flüssiges, aus Soja-Bohnen isoliertes Phospholipidgemisch enthält, das mindestens 40 Gew.% 1,2-Diacylglycero-3-Phosphatidylcholin sowie übliche Begleitphospholipide und Öle aufweist.

15. Verfahren zur Herstellung einer phospholipidischen Zusammensetzung nach einem der vorangehenden Ansprüche, dadurch gekennzeichnet, daß das mindestens eine Phospholipid in einem Lösungsmittel oder Lösungsmittelgemisch gelöst oder dispergiert wird, daß die Lösung bzw. Dispersion des Phospholipids in eine Lösung oder Dispersion des Stabilisators eingebracht wird und daß die so hergestellte Mischung anschließend schonend getrocknet, insbesondere sprühgetrocknet oder gefriergetrocknet, wird.

16. Verfahren nach Anspruch 15, dadurch gekennzeichnet, daß als Lösungsmittel bzw. Lösungsmittelgemisch Wasser und/oder ein Alkohol, vorzugsweise Ethanol, Propanol-1 und/oder Propanol-2, verwendet wird.

17. Verfahren nach Anspruch 15 oder 16, dadurch gekennzeichnet, daß eine alkoholische Lösung des Phospholipids hergestellt wird und daß die alkoholische Lösung in eine wäßrige Dispersion oder in eine wäßrige Lösung des Stabilisators eingerührt oder injiziert wird.

18. Verfahren nach Anspruch 17, dadurch gekennzeichnet, daß die alkoholische Phospholipidlösung 70 Gew.% bis 85 Gew.% Phospholipid und 30 Gew.% bis 15 Gew.% des Alkohol enthält.

19. Verfahren nach Anspruch 15 oder 16, dadurch gekennzeichnet, daß eine wäßrige Dispersion des Phospholipids hergestellt wird und daß die wäßrige Dispersion in die wäßrige Dispersion des Stabilisators eingerührt oder injiziert wird.

20. Verfahren nach Anspruch 19, dadurch gekennzeichnet, daß die wäßrige Dispersion 5 Gew.% bis 20 Gew.%, insbesondere 8 Gew.% bis 15 Gew.%, des Phospholipids enthält.

21. Verfahren nach einem der Ansprüche 15 bis 20, dadurch gekennzeichnet, daß eine wäßrige Dispersion oder Lösung des Stabilisators hergestellt wird, wobei die Stabilisatordispersion bzw. Stabilisatorlösung 10 Gew.% bis 30 Gew.% des Stabilisators enthält, und daß durch Variation der Menge der Stabilisatordispersion bzw. -lösung und/oder der Phospholipidlösung bzw. - dispersion in der Mischung ein Gewichtsverhältnis von Phospholipid zu Stabilisator zwischen 1:0,08 bis 1:0,002, insbesondere zwischen 1:0,04 und 1:0,003, eingestellt wird.

22. Verfahren zur Herstellung der Zusammensetzung nach einem der Ansprüche 1 bis 15, dadurch gekennzeichnet, daß ein flüssiges Phospholipid bzw. Phospholipidgemisch hergestellt wird, derart, daß das Phospholipid bzw. Phospholipidgemisch in einem Öl aufgenommen wird, und daß das in dem Öl aufgenommene Phospholipid bzw. Phospholipidgemisch mit dem Stabilisator, einer Stabilisatorlösung und/oder einer Stabilisatordispersion vermischt wird.

23. Verwendung der Zusammensetzung nach einem der vorangehenden Ansprüche zur Herstellung von kosmetischen Mitteln.

24. Verwendung der Zusammensetzung nach einem der vorangehenden Ansprüche zur Herstellung von pharmazeutischen Mitteln.
